(19) Europäisches Patentamt
European Patent Office
Office européen des brevets

(11) Numéro de publication: **0 383 690 A1**

(12) **DEMANDE DE BREVET EUROPEEN**

(21) Numéro de dépôt: **90400416.5**

(22) Date de dépôt: **15.02.90**

(51) Int. Cl.[5]: **C07D 209/42, C07D 213/82, C07D 215/54, C07D 211/60, C07C 237/06, A61K 31/40, A61K 31/44, A61K 31/47, A61K 31/445, A61K 31/195**

(30) Priorité: **17.02.89 FR 8902093**

(43) Date de publication de la demande: **22.08.90 Bulletin 90/34**

(84) Etats contractants désignés: **CH DE FR GB IT LI NL**

(71) Demandeur: **ROUSSEL-UCLAF**
**35, boulevard des Invalides**
**F-75007 Paris(FR)**

(72) Inventeur: **Gasc, Jean-Claude**
**6, rue Georges Lyssandre**
**F-93140 Bondy(FR)**
Inventeur: **Humbert, Daniel**
**15, rue Gaston Charle**
**F-94120 Fontenay sous Bois(FR)**
Inventeur: **Vekens, Mario**
**9, Square Régnault**
**F-92400 Courbevoie(FR)**

(74) Mandataire: **Tonnellier, Marie-José et al**
**111, route de Noisy B.P. no 9**
**F-93230 Romainville(FR)**

(54) **Nouveaux dérivés de l'acide 2-amino pentanedioique, leur procédé et les intermédiaires de préparation, leur application comme médicaments et les compositons les renfermant.**

(57) L'invention a pour objet les composés de formule (I) :

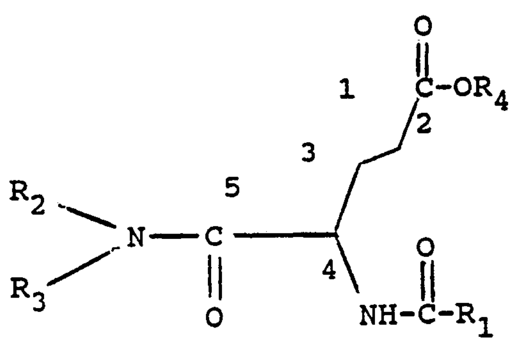

(I)

dans lesquels $\underline{R_1}$ représente :
- ou bien un radical aryle, éventuellement substiuté,
- ou bien un radical hétérocyclique,
- ou bien un radical arylalkyle,

et soit $\underline{R_2}$ représente : - ou bien un radical

EP 0 383 690 A1

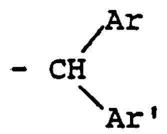

dans lesquels Ar et Ar′ représentent un radical aryle éventuellement substitué ou un radical hétérocyclique,
- ou bien un radical

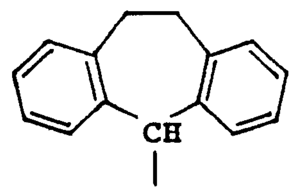

éventuellement substitué
et $R_3$ représente un atome d'hydrogène,
soit $R_2$ et $R_3$ forment ensemble avec l'atome d'azote, auquel ils sont liés un cycle à 5, 6 ou 7 chaînons éventuellement substitué,
et $R_4$ représente un atome d'hydrogène, un radical alkyle, un radical arylalkyle éventuellement substitué ou un radical

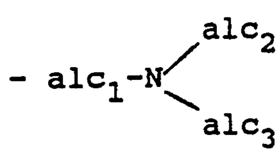

leur procédé et intermédiaires de préparation, leur application comme médicaments et les compositions les renfermant.

**Nouveaux dérivés de l'acide 2-amino pentanedioïque leur procédé et les intermédiaires de préparation, leur application comme médicaments et les compositions les renfermant.**

La présente invention concerne de nouveaux dérivés de l'acide 2-amino pentanedioïque, leur procédé et les intermédiaires de préparation, leur application comme médicament et les compositions les renfermant.

L'invention a pour objet les composés de formule (I) :

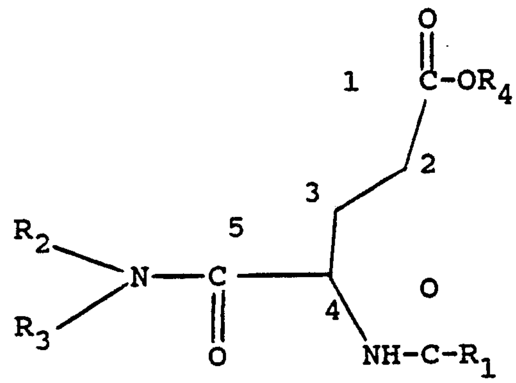

(I)

dans lesquels $R_1$ représente :

- ou bien un radical aryle renfermant jusqu'à 14 atomes de carbone éventuellement substitué par un ou plusieurs substituants choisis dans le groupe constitué par les atomes d'halogène, les radicaux alkyle ou alkyloxy renfermant jusqu'à 8 atomes de carbone, les radicaux dialkylamino renfermant jusqu'à 16 atomes de carbone et les radicaux $CF_3$, cyano ou nitro,
- ou bien un radical hétérocyclique choisi dans le groupe constitué par les radicaux indolyle, pyridyle, pipéridinyle, quinolyle et thiazolyle,
- ou bien un radical arylalkyle renfermant jusqu'à 18 atomes de carbone éventuellement substitué sur le noyau aryle par un ou plusieurs des substituants indiqués ci-dessus lorsque $R_1$ représente un radical aryle

et

soit $R_2$ représente :

- ou bien un radical

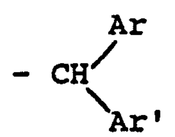

dans lesquels Ar et Ar′ identiques ou différents représentent un radical aryle renfermant jusqu'à 14 atomes de carbone éventuellement substitué par un ou plusieurs des substituants indiqués ci-dessus pour $R_1$, ou un radical hétérocyclique choisi dans le groupe constitué par les radicaux pyridyle, indolyle et thiazolyle,

- ou bien un radical

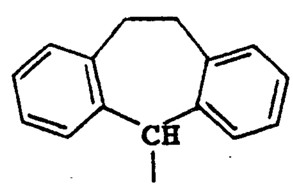

dans lequel les noyaux phényle peuvent être substitués par un ou plusieurs des substituants indiqués ci-dessus pour $R_1$,

et $R_3$ représente un atome d'hydrogène,

soit $R_2$ et $R_3$ forment ensemble avec l'atome d'azote, auquel ils sont liés un cycle carboné à 5, 6 ou 7

chaînons contenant éventuellement un atome d'oxygène ou un second atome d'azote éventuellement substitué par un ou plusieurs des radicaux choisis dans le groupe constitué par les radicaux alkyle renfermant jusqu'à 8 atomes de carbone, les radicaux aryle renfermant jusqu'à 14 atomes de carbone, les radicaux

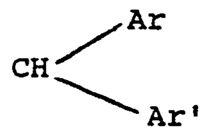

dans lesquels Ar et Ar′ conservent la même signification que précédemment,
et $R_4$ représente un atome d'hydrogène, un radical alkyle linéaire, ramifié ou cyclique renfermant jusqu'à 8 atomes de carbone, un radical arylalkyle renfermant jusqu'à 18 atomes de carbone éventuellement substitué par un ou plusieurs des substituants indiqués ci-dessus pour $R_1$, ou un radical

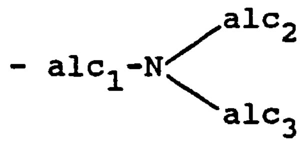

dans lesquels $alc_1$ représente un radical polyméthylène renfer mant jusqu'à 8 atomes de carbone et $alc_2$ et $alc_3$ identiques ou différents représentent un radical alkyle linéaire, ramifié ou cyclique renfermant jusqu'à 8 atomes de carbone, sous toutes leurs formes isomères possibles ainsi que leurs mélanges.

Les composés de l'invention possèdent un ou plusieurs carbones asymétriques, ils peuvent donc exister sous forme racémique ou sous une forme énantiomère, ils possèdent tous un carbone asymétrique en position 4, et peuvent également posséder d'autres carbones asymétriques selon les valeurs des substituants.

Lorsque $R_1$ représente un radical aryle, il s'agit de préférence du radical phényle éventuellement substitué. Par un atome d'halogène, on entend de préférence les atomes de chlore et de brome.

Lorsque $R_1$ représente un radical aryle substitué par un radical alkyle, il s'agit de préférence d'un radical phényle substitué par un radical méthyle, éthyle, n-propyle ou isopropyle.

Lorsque $R_1$ représente un radical aryle substitué par un radical alkyloxy, il s'agit de préférence d'un radical phényle substitué par un radical méthoxy, éthoxy ou n-propoxy.

Lorsque $R_1$ représente un radical aryle substitué par un radical dialkylamino, il s'agit de préférence d'un radical phényle substitué par un radical diméthylamino ou diéthylamino.

Lorsque $R_1$ représente un radical arylalkyle, il s'agit de préférence du radical benzyle, d'un radical $-CH_2-CH_2-C_6H_5$, ou $(CH_2)_3C_6H_5$ éventuellement substitué par l'un des substituants déjà indiqués, ou encore du radical cinnamique.

Ar et Ar′ représentent de préférence un radical phényle éventuellement substitué.

Lorsque $R_2$ et $R_3$ forment ensemble avec l'atome d'azote auquel ils sont liés un cycle carboné, il s'agit de préférence des radicaux :

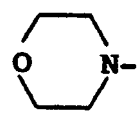 ou 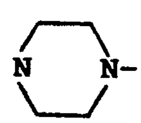

eventuellement substitué et tout spécialement des radicaux :

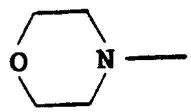

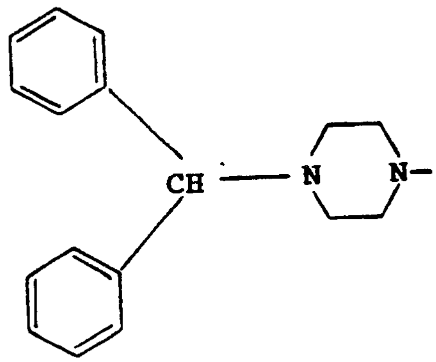

ou

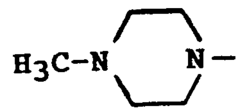

Lorsque $R_4$ représente un radical alkyle, il s'agit de préférence d'un radical méthyle, éthyle, n-propyle, isopropyle ou n-butyle.

Lorsque $R_4$ représente un radical arylalkyle, il s'agit de préférence du radical benzyle, éventuellement substitué.

Lorsque $R_4$ représente un radical :

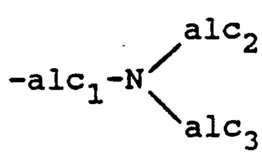

il s'agit de préférence du radical :

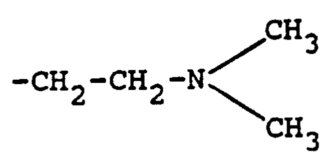

ou :

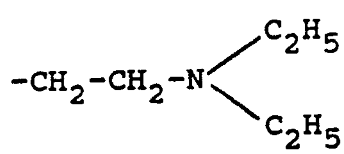

ou :

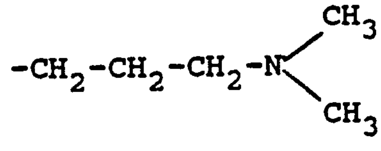

ou :

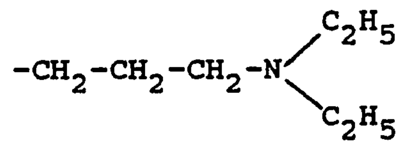

Parmi les composés préférés de l'invention, on peut citer les composés dans lesquels $R_1$ représente un radical indolyle, ceux dans lesquels $R_2$ représente un radical :

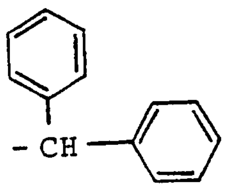

dans lequel les noyaux phényles sont éventuellement substitués par un ou plusieurs des substituants énumérés ci-dessus pour $R_1$, et tout spécialement les composés dans lesquels $R_2$ représente le radical :

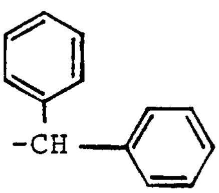

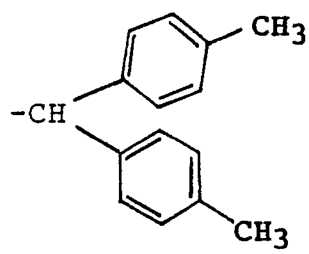

,

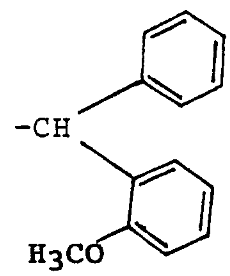

ou :

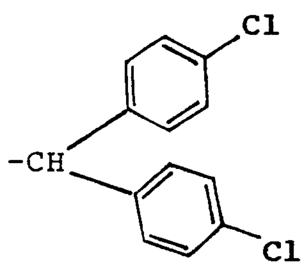

On peut également citer les composés de formule (I) dans lesquels $R_2$ représente un radical :

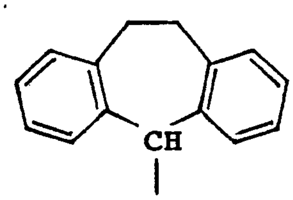

ainsi que ceux dans lesquels $R_4$ représente un atome d'hydrogène.

Parmi les composés préférés de l'invention, on peut citer les composés de configuration S au niveau du carbone en 4.

L'invention a naturellement tout particulièrement pour objet les composés dont la préparation est donnée ci-après dans la partie expérimentale et tout spécialement les composés des exemples 2, 4, 9 et 13.

Les composés de formule (I) sont des agonistes ou des antagonistes de la cholecystokinine dont des sites de liaison ont été mis en évidence aux niveaux central et périphérique. La cholecystokinine est un peptide largement distribué dans le cerveau, notamment dans le cortex, le striatum, l'hippocampe, le tegmentum ventral, le septum et l'hypothalamus.

La cholecystokinine est également secrétée au niveau périphérique par l'intestin grêle, son action se manifeste notamment par la stimulation de la contraction vésiculaire, une augmentation de la secrétion biliaire, le contrôle de la secrétion enzymatique pancréatique, une action sur les contractions gastriques, une action sur la motilité intestinale. Elle pourrait agir dans certains cas sur la pression artérielle et influencer les systèmes immunitaires.

La cholecystokinine coexiste dans certains neurones centraux avec la dopamine. Elle intervient également dans des mécanismes impliquant l'acéthylcholine, le gaba, la sérotonine, les opioïdes, la somatostatine, la substance P et des canaux ioniques.

Son administration provoque des modifications physiologiques : ptose palpébrale, hypothermie, hyperglycémie, catalepsie, et comportementales : hypolocomotricité, diminution de l'exploration, analgésie, action dans les apprentissages, modification du comportement sexuel et satiété.

Selon les doses, elle se comporte comme un agoniste ou un antagoniste dopaminergique.

Les composés de formule (I) peuvent donc être utilisés comme médicaments dans le traitement de certains troubles du comportement alimentaire, de l'obésité, dans des troubles des comportements, émotionnel, sexuel et mnésique, dans la schizophrénie et dans divers troubles de la sphère gastrointestinale.

L'invention a donc pour objet à titre de médicaments les composés de formule (I), et tout particulièrement les composés dont la préparation est donnée ci-après dans la partie expérimentale, notamment les produits des exemples 2, 4, 9 et 13.

La posologie, variable selon le produit utilisé et l'affection en cause peut s'échelonner, par exemple, entre 0,05 mg et 100 mg par jour chez l'adulte par voie orale.

La présente demande a encore pour objet les compositions pharmaceutiques qui contiennent, à titre de principe actif, au moins un des médicaments précités. Ces compositions sont réalisées de façon à pouvoir être administrées par la voie digestive ou parentérale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation, caractérisé en ce que l'on soumet un composé de formule (II) :

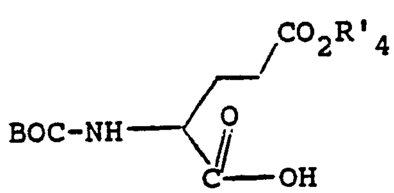

(II)

dans laquelle BOC représente le radical (1,1-diméthyl éthoxy) carbonyle et $R'_4$ a la même signification que $R_4$ à l'exception de la valeur hydrogène, à l'action d'un composé de formule (III) :

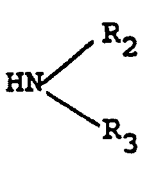

(III)

dans laquelle $R_2$ et $R_3$ conservent leur signification précédente pour obtenir le composé de formule (IV) :

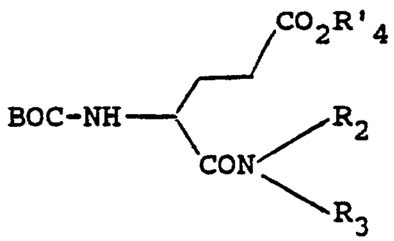

(IV)

que l'on soumet à l'action d'un agent de déblocage de la fonction amine, puis soumet le produit résultant à l'action d'un acide ou dérivé d'acide de formule (V) :

$R_1CO_2H$ (V)

dans laquelle $R_1$ conserve sa signification précédente pour obtenir le composé de formule ($I_A$) :

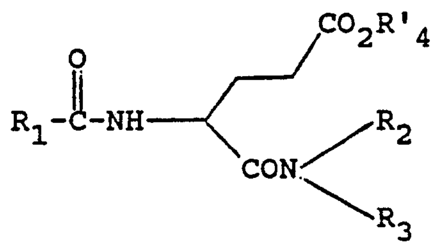

($I_A$)

que l'on soumet si désiré à l'action d'un agent de clivage de la fonction acide pour obtenir le composé de formule ($I_B$) :

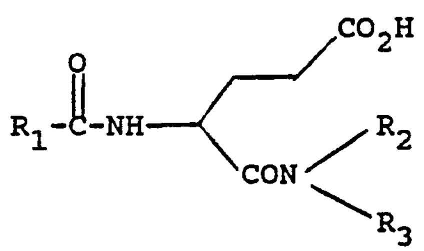

($I_B$)

Dans un mode de réalisation préférée du procédé de l'invention :

- le composé de formule (III) est utilisé sous forme de sel, par exemple de chlorhydrate,
- dans le composé de formule (II) $R'_4$ représente un radical benzyle,
- l'agent de déblocage de la fonction amine est un acide fort, comme par exemple l'acide chlorhydrique, ou l'acide trifluoroacétique et l'on opère au sein d'un solvant organique comme le chlorure de méthylène ou l'acétate d'éthyle,
- l'acide de formule (V) est utilisé sous forme d'acide, de chlorure d'acide ou d'anhydride d'acide,
- l'agent de déblocage de la fontion acide est l'hydrogène en présence de palladium ou une base telle que la soude, la potasse, le carbonate de potassium, l'hydroxyde de lithium, et l'on opère de préférence au sein d'un solvant comme l'éthanol, le dioxanne, le tétrahydrofuranne, le méthanol, le diméthylformamide à une température comprise entre 0° C environ et la température de reflux du solvant utilisé.

L'invention a également pour objet une variante du procédé précédent, caractérisé en ce que l'on soumet un composé de formule (VI) :

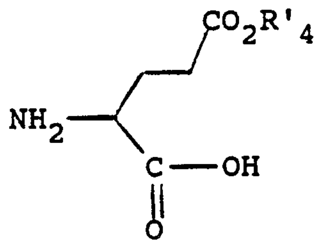

(VI)

dans laquelle $R'_4$ a la signification indiquée précédemment à l'action d'un acide ou d'un dérivé d'acide de formule (V) :

$R_1CO_2H$ (V)

dans laquelle $R_1$ a la signification indiquée précédemment pour obtenir le composé de formule (VII) :

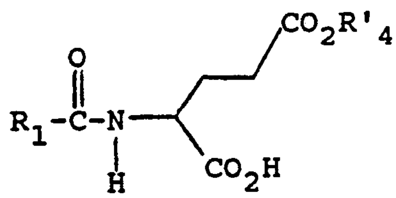

(VII)

que l'on soumet à l'action d'un composé de formule (III) :

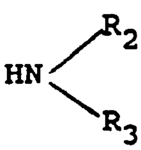

(III)

dans laquelle $R_2$ et $R_3$ ont la signification indiquée précédemment pour obtenir le composé de formule ($I_A$) correspondant que l'on soumet si désiré, à l'action d'un agent de clivage de la fonction acide pour obtenir le composé de formule ($I_B$), ($I_A$) et ($I_B$) étant définis comme précédemment.

Dans un mode de réalisation préféré du procédé de l'invention :

- l'acide de formule (V) est utilisé sous forme de chlorure d'acide,
- dans le composé de formule (VI) $R'_4$ représente un radical benzyle.

Les produits de formule (IV) et (VII) obtenus lors de la mise en oeuvre du procédé de l'invention sont des produits nouveaux et sont en eux-mêmes un objet de la présente invention.

Les exemples suivants illustrent l'invention sans toutefois la limiter.

**EXEMPLE 1 : Acide (±) 4-[(benzoyl) amino] 5-[(diphénylméthyl) amino] 5-oxo pentanoïque**

STADE A : Acide (±) 4-[[(1,1-diméthyl éthoxy) carbonyl] amino] 5-[(diphénylméthyl) amino] 5-oxo pentanoïque.

A une suspension composée de :

- 17,62 g de chlorhydrate de alpha-phényl benzène-méthanamine,
- 24,5 g de N-[(1,1-diméthyl éthoxy) carbonyl] L-5-glutamate de phénylméthyle,
- 10,5 g de N-hydroxybenzotriazole,
- 30,7 g de chlorhydrate de 1-[3-(diméthylamino) propyl] 3-éthyl carbodiimide,
- 350 $cm^3$ de chlorure de méthylène,

on ajoute goutte à goutte à 5 °C sous agitation :

- 20,45 $cm^3$ de N-éthyl morpholine.

On laisse revenir à température ambiante et on agite 18 heures. On lave le milieu réactionnel successivement avec 200 $cm^3$ d'acide chlorhydrique, 200 $cm^3$ d'une solution 1N de carbonate de sodium et

100 $cm^3$ d'eau. On sèche la phase organique sur sulfate de magnésium et on concentre le solvant.
On obtient 36,3 g d'un solide blanc.
F = 128°
Rdt = 90,2 %.

STADE B : (±) 4-amino 5-[(diphénylméthyl) amino] 5-oxo pentanoate de phénylméthyle.

On dissout 36,3 g du dérivé obtenu précédemment dans 700 $cm^3$ d'acétate d'éthyle.

On refroidit à 5° C par un bain de glace et on fait passer un courant d'acide chlorhydrique dans la solution pendant une heure. On élimine le solvant sous pression réduite. On reprend le résidu obtenu dans 500 $cm^3$ d'éther éthylique. On filtre le produit et on obtient 30,8 g du chlorhydrate attendu.
F = 105°.
Rdt = 97,4 %.

STADE C : (±) 4-[(benzoyl) amino] 5-[(diphénylméthyl) amino] 5-oxo pentanoate de phénylméthyle.

A une suspension préparée à partir de :
- 2 g du chlorhydrate préparé ci-dessus,
- 0,55 g d'acide benzoïque,
- 0,91 g d'hydrate d'hydroxy benzotriazole,
- 1,72 g de chlorhydrate de 1-[3-(diméthylamino) propyl] 3-éthyl carbodiimide,
- 20 $cm^3$ de chlorure de méthylène,

on ajoute goutte à goutte, sous agitation et à 5° C :
- 1,7 $cm^3$ de N-éthylmorpholine.

On laisse revenir à température ambiante et on agite pendant 18 heures. On lave la phase organique successivement par 10 $cm^3$ d'acide chlorhydrique N, 10 $cm^3$ d'une solution normale de carbonate de sodium et 2 fois 10 $cm^3$ d'eau. On sèche sur sulfate de magnésium, puis on élimine le solvant. On obtient 2,4 g d'un solide jaunâtre.
F = 115°
Rdt = 93,8 %.

STADE D : Acide (±) 4-[(benzoyl) amino] 5-[(diphénylméthyl) amino] 5-oxo pentanoïque.

On hydrogénolyse 2,1 g de l'ester benzylique obtenu précédemment en présence de 500 mg de charbon palladié à 10 % dans un mélange de 50 $cm^3$ d'éthanol et 10 $cm^3$ d'acide acétique. Après absorption de la quantité souhaitée d'hydrogène, on filtre le catalyseur. On élimine les solvants sous pression réduite, on reprend le résidu obtenu dans 50 $cm^3$ d'éther.
On obtient un solide blanc.
F = 118° C.

**EXEMPLE 2 : Acide (S)-4-[[(1H-indol-2-yl) carbonyl] amino] 5-[[bis(4-méthyl phényl) méthyl] amino] 5-oxo pentanoïque.**

STADE A : Acide (S)-2-[[(1H-indol-2-yl) carbonyl] amino] pentanedioate de 5-(phénylméthyle).

On met en suspension, sous azote 4,27 g de L-5-glutamate de phénylméthyle, dans 50 $cm^3$ d'acétone, puis on ajoute 22,5 mmoles de carbonate de sodium, sous forme d'une solution 1N dans l'eau. On agite une demi-heure, puis on refroidit entre 5 et 10° C et on ajoute goutte à goutte, une solution de 2,9 g de chlorure de l'acide 1H-indol-2-carboxylique dans 50 $cm^3$ d'acétone.

A la fin de l'addition, on laisse revenir à température ambiante, et on garde sous agitation pendant 1 nuit.

On verse dans 200 $cm^3$ d'eau puis on acidifie par de l'acide chlorhydrique concentré. On extrait à l'acétate d'éthyle et on lave cette phase organique avec une solution saturée de chlorure de sodium puis on

la sèche sur du sulfate de magnésium. On élimine le solvant sous pression réduite. Le résidu huileux obtenu est purifié par chromatographie sur silice puis recristallisé dans l'éther. On obtient 6,7 g d'un solide beige.
F = 144° C.

STADE B : (S)-4-[[(1H-indol-2-yl) carbonyl) amino] 5-[[bis(4-méthyl phényl) méthyl] amino] 5-oxo pentanoate de phénylméthyle.

On introduit successivement 1,9 g du produit obtenu au stade précédent, 100 $cm^3$ de chlorure de méthylène, 1,01 g d'hydrate d'hydroxy benzotriazole, 1,42 g de chlorhydrate de 1-[3-(diméthylamino) propyl] 3-éthyl carbodiimide, et 1,06 g de la bis-4,4 diméthyl benzhydrylamine.

On laisse agiter une nuit puis on lave par un volume d'acide chlorhydrique 1N, un volume d'une solution saturée en bicarbonate de sodium et par un volume d'eau. Après avoir séché la phase organique sur du sulfate de magnésium, on filtre et on élimine le solvant sous pression réduite. On obtient 2,6 g du produit recherché, fondant à 146 ≈ 150° C.

STADE C : Acide (S)-4-[[(1H-indol-2-yl) carbonyl] amino] 5-[[bis(4-méthyl phényl) méthyl] amino] 5-oxo pentanoïque.

On fait passer un courant d'hydrogène en présence de 500 mg de palladium sur charbon actif à 10% dans une solution renfermant 2,56 g de produit obtenu au stade B, 250 $cm^3$ d'éthanol absolu et 5 $cm^3$ d'acide acétique. On filtre le catalyseur. Le solvant est éliminé et le résidu obtenu est cristallisé dans l'éthanol. On obtient 2,2 g du produit recherché F > 260° C.

**EXEMPLE 3 :**

En opérant comme précédemment, on a obtenu l'ester benzylique de l'acide (S)-5-[[bis(4-chloro phényl) méthyl] amino] 5-oxo 4-[[(1H-indol-2-yl) carbonyl] amino] pentanoïque.
F = 207° C.

**EXEMPLE 4 : Acide (S)-5-[[bis(4-chloro phényl) méthyl] amino] 5-oxo 4-[[(1H-indol-2-yl) carbonyl] amino] pentanoïque.**

On introduit, entre 5° C et 10° C, 2,1 g d'ester benzylique de l'acide (S)-5-[[bis(4-chloro phényl) méthyl] amino] 5-oxo 4-[[(1H-indol-2-yl) carbonyl] amino] pentanoïque, dans 30 $cm^3$ d'éthanol à 90 %. On obtient une suspension à laquelle on ajoute 6,8 $cm^3$ de soude 1N. On laisse revenir à température ambiante et on agite 16 heures. On filtre le léger insoluble. Le solvant est éliminé et le résidu obtenu est repris dans 20 $cm^3$ d'eau. La solution résultante refroidie à 0°/5° C est acidifiée par de l'acide chlorhydrique concentré. On obtient un précipité incolore que l'on filtre et lave à l'eau.

Le produit est séché sous pression réduite. On obtient ainsi 1,6 g d'un solide incolore.
Rendement = 89,7 %.

Le composé est recristallisé dans un mélange méthanol/chloroforme. Après filtration on obtient des cristaux incolores.
F > 260° C.

| Microanalyse : | | | | |
|---|---|---|---|---|
| Calculé :<br>Trouvé : | C% 61,84<br>61,8 | H% 4,42<br>4,3 | N% 8,01<br>8,0 | Cl% 13,52<br>13,5 |
| $[Alpha]_D$ + 0,967°, (C = 1,55 % dans le diméthyl formamide). | | | | |

En opérant comme indiqué dans les exemples ci-dessus, on a obtenu les produits suivants :

EXEMPLE 5 : Acide 5-[(diphénylméthyl) amino] 4-[[(1H-indol-2-yl) carbonyl] amino] 5-oxo pentanoïque.
F = 258 ≈ 60° C.

EXEMPLE 6 : (S) 4-[[(1H-indol-2-yl) carbonyl] amino] 5-[[bis-(4-méthoxy phényl) méthyl] amino] 5-oxo pentanoate de phénylméthyle.
F = 187° C.

EXEMPLE 7 : Acide (S)-4-[[(1H-indol-2-yl) carbonyl] amino] 5-[[bis(4-méthoxy phényl) méthyl] amino] 5-oxo pentanoïque.
F = 157° C.

EXEMPLE 8 : (4S)-4-[[(1H-indol-2-yl) carbonyl] amino] 5-[[(2-méthoxy phényl) phénylméthyl] amino] 5-oxo pentanoate de phénylméthyle.
F = 148° C.

EXEMPLE 9 : Acide (4S)-4-[[(1H-indol-2-yl) carbonyl] amino] 5-[[(2-méthoxy phényl) phénylméthyl] amino] 5-oxo pentanoïque.
F = 228° C.

EXEMPLE 10 : (S)-4-[(3,4-dichloro benzoyl) amino] 5-[(diphénylméthyl) amino] 5-oxo pentanoate de phénylméthyle.
F = 164° C.

EXEMPLE 11 : Acide (S)-4-[(3,4-dichloro benzoyl) amino] 5-[(diphénylméthyl) amino] 5-oxo pentanoïque.
F = 204° C.

EXEMPLE 12 : Acide (S)-5-[(diphénylméthyl) amino] 4-[[(1H-indol-3-yl) carbonyl] amino] 5-oxo pentanoïque.
F = 141° C.
Le produit a été préparé à partir de l'ester benzylique correspondant. F = 190° C.

EXEMPLE 13 : Acide (S)-5-[(10,11-dihydro dibenzo[a,d]cyclohepten-5-yl) amino] 4-[[(1H-indol-2-yl) carbonyl] amino] 5-oxo pentanoïque.
F > 260° C.

Le produit a été préparé à partir de l'ester benzylique correspondant.
F = 170 ≈ 200° C

EXEMPLE 14 : Acide (S)-5-[(diphénylméthyl) amino] 4-[[(1H-indol-2-yl) carbonyl] amino] 5-oxo pentanoïque.
F ≈ 200° C.
Le produit a été préparé à partir de l'ester benzylique correspondant.
F = 168° C.

EXEMPLE 15 : Acide (S)-4-[(4-bromo benzoyl) amino] 5-[(diphénylméthyl) amino] 5-oxo pentanoïque.
F = 215° C.
Le produit a été préparé à partir de l'ester benzylique correspondant.
F = 150° C.

EXEMPLE 16 : Acide (S)-5-[(diphénylméthyl) amino] 5-oxo 4-[[(3-pyridyl) carbonyl] amino] pentanoïque.
F = 220° C.
Le produit a été préparé à partir de l'ester benzylique correspondant.
F ≈ 100° C.

EXEMPLE 17 : Acide (S)-5-[(diphénylméthyl) amino] 4-[(4-méthoxy benzoyl) amino] 5-oxo pentanoïque.
F = 161° C.
Le produit a été préparé à partir de l'ester benzylique correspondant.
F = 158° C.

EXEMPLE 18 : Acide (S)-5-[(diphénylméthyl) amino] 5-oxo 4-[[(3-quinolyl) carbonyl] amino] pentanoïque.
F = 240° C.
Le produit a été préparé à partir de l'ester benzylique correspondant.
F = 240° C.

EXEMPLE 19 : Acide (S)-5-[(diphénylméthyl) amino] 4-[(4-nitro benzoyl) amino] 5-oxo pentanoïque.

F = 219 °C.
Le produit a été préparé à partir de l'ester benzylique correspondant. F = 137 °C.

**EXEMPLE 20 : Acide (4S)-5-[(diphénylméthyl) amino] 5-oxo 4-[[(3-piperidinyl) carbonyl] amino] pentanoïque.**
F = 166 °C.

**EXEMPLE 21 : Acide (S)-4-[[(1H-indol-2-yl) carbonyl] amino] 5-oxo 5-(1-pipérazinyl) pentanoïque.**

**EXEMPLE 22 : Acide (S)-5-[4-(diphénylméthyl) 1-pipérazinyl] 4-[[(1H-indol-2-yl) carbonyl] amino] 5-oxo pentanoïque.**

**EXEMPLE 23 : Acide (4S)-4-[[(1H-indol-2-yl) carbonyl] amino] 5-oxo 5-[phényl (3-pyridyl) méthylamino] pentanoïque.**

**EXEMPLE 24 : (S)-5-[(diphénylméthyl) amino] 4-[[(1H-indol-2-yl) carbonyl] amino] 5-oxo pentanoate de 2-(diméthylamino) éthyle.**

## FORMES PHARMACEUTIQUES

### EXEMPLE 25 : Comprimés

On a préparé des comprimés répondant à la formule suivante :

| | |
|---|---|
| - Produit de l'exemple 2 | 20 mg |
| - Excipient q.s. pour un comprimé terminé à | 300 mg |
| (Détail de l'excipient : lactose, amidon de blé, amidon traité, amidon de riz, stéarate de magnésium, talc). | |

### EXEMPLE 25 : Gélules

On a préparé des gélules répondant à la formule suivante :

| | |
|---|---|
| - Produit de l'exemple 9 | 50 mg |
| - Excipient q.s. pour une gélule terminée à | 300 mg |
| (Détail de l'excipient : talc, stéarate de magnésium, aérosil). | |

## ETUDE BIOLOGIQUE

### 1) Récepteurs centraux.

On prélève les cortex de 20 rats mâles pesant de 150 à 200 g, ces cortex sont broyés au Polytron dans du sucrose 0,32 M.

Après centrifugation le surnageant est recueilli et centrifugé.

Les culots sont remis en suspension dans 120 ml tampon HEPES pH 7,4 (Hepes 10 mM, NaCl 130 mM, $MgCl_2$, $6H_2O$ mM, bacitracine 250 mg/l, PMSF 1 mg/l), et recentrifugés.

Les culots sont repris dans 120 ml de tampon Hepes pH 7,4 et centrifugés de nouveau à 30000 g pendant 30 mn.

Les culots ainsi obtenus sont repris par 500 ml de tampon Hepes pH 7,4, ce qui permet d'obtenir 240

aliquotes de 2 ml d'homogénat.

L'incubation est faite à 25° C, 30 mn en présence de 0,5 nM de 3H CCK8 et du produit à tester (10000 micromoles pour 1 dose, ou avec une gamme de 7 doses) ou de CCK8 froide ($10^{-6}$M), qui est le produit de référence.

Après retour à 0° C des aliquotes d'homogénat, ceux-ci sont filtrés sur filtres Whatman GF/B et les filtres lavés par 3x5 ml d'un tampon Tris HCl 50 mM pH 7,4.

Les résultats sont exprimés en $CI_{50}$ : concentration nécessaire pour inhiber de 50 % la radioactivité spécifique fixée.

2) Récepteurs périphériques.

Les pancréas de 3 rats mâles, 150-200 g, sont prélevés et broyés au Polytron (4 broyages, vitesse 3, avec un intervalle de 10 secondes entre les broyages) ; l'homogénat est filtré sur une gaze, puis centrifugé à 30000 g pendant 30 minutes.

Les culots obtenus sont repris par 400 volumes (≃ 600 ml) de tampon Tris 50 mM HCl pH 7,4, contenant BSA 2 g/l, bacitracine 0,1 mM, Mg $Cl_2$ 5 mM, dithiothreitol 5 mM.

Des aliquotes d'homogénat de 2 ml sont incubés à 25° C 60 mn, en présence de 0,2 nM 3H CCK8 et du produit à tester (10000 micromoles pour 1 dose, ou avec 1 gamme de 7 doses) et de CCK8 froide ($10^{-6}$ M) qui est le produit de référence.

Après retour à 0° C, les aliquotes sont filtrés sur filtres Whatman GF/B, prélavés dans une solution de polyéthylène imine à 0,05 %. Lavage par 3x5 ml de tampon Tris HCl 50 mM pH 7,4.

Les résultats sont exprimés en $CI_{50}$ : concentration nécessaire, pour inhiber de 50 % la radioactivité spécifique fixée.

($CI_{50}$ en nanomoles).

| | CCK | CCK |
|---|---|---|
| | Périphérique | Central |
| Exemple 2 | 53 | 700 |
| Exemple 4 | 96 | > 10 000 |
| Exemple 9 | 32 | 682 |
| Exemple 13 | 1 660 | 2 660 |

**Action sur la prise alimentaire chez le rat.**

Technique :

Les essais sont réalisés sur des lots de 5 rats de 250 ± 10 g dans les conditions suivantes : les animaux sont placés individuellement dans des cages ayant une mangeoire décrite par FREGLY (J. Appl. Physiol., 1960, 15, 539) qui présente l'avantage d'éviter le gaspillage de nourriture constituée par de la provende pulvérisée. Les rats sont habitués à prendre leur ration quotidienne en 5 h consécutives, l'eau de boisson étant offerte ad libitum.

Les quantités de nourriture ingérées sont déterminées individuellement par la pesée des mangeoires. Les consommations sont suivies d'heure en heure pendant 5 heures après l'administration de 10 mg/kg i.p. du composé. Les quantités consommées sont exprimées en g/100 g de poids corporel, par heure.

Les moyennes sont comparées à celles obtenues chez des animaux témoins par un test de DUNNETT.

Résultats :

A la dose de 10 mg/kg par voie intrapéritonéale, le produit de l'exemple 9 manifeste une activité anorexigène.

Il réduit significativement de plus de 50 % la consommation alimentaire des animaux traités par rapport à celle des animaux témoins.

A cette dose, le produit de l'exemple 2 réduit de 15 %, de façon statistiquement non significative, la consommation alimentaire.

## Action sur l'iléon isolé de cobaye.

Méthode :

L'essai est réalisé sur des fragments d'iléon de cobaye mâles, placés sous une tension de 1 g dans une solution de Krebs aérée au carbogène et maintenue à 37° C. Les contractions sont enregistrées à l'aide d'un microdynamomètre relié à un polygraphe.

L'iléon est laissé au repos pendant 30 minutes, puis la CCK8 est ajoutée dans le bain à la concentration de $1\text{-}10^{-8}$M. On rince. Le produit à étudier est ajouté dans le bain, laissé en contact 1 minute avec l'organe, puis du CCK8 ($1\text{-}10^{-8}$M) est ajoutée dans le bain. L'antagonisme éventuel est exprimé en comparant les contractions engendrées par la CCK8 avant et après contact du produit à tester.

Le produit de l'exemple 9 à une dose de $10^{-6}$ M présente une activité antagoniste intéressante.

## Revendications

1) Les composés de formule (I) :

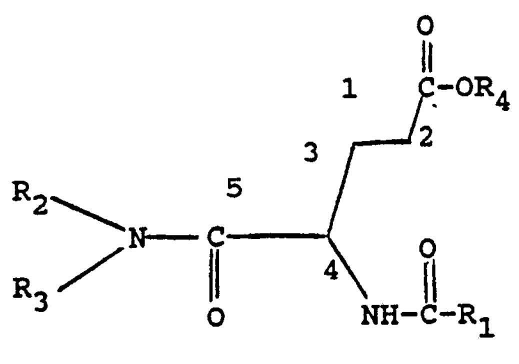

(I)

dans lesquels $R_1$ représente :
- ou bien un radical aryle renfermant jusqu'à 14 atomes de carbone éventuellement substitué par un ou plusieurs substituants choisis dans le groupe constitué par les atomes d'halogène, les radicaux alkyle ou alkyloxy renfermant jusqu'à 8 atomes de carbone, les radicaux dialkylamino renfermant jusqu'à 16 atomes de carbone et les radicaux $CF_3$, cyano ou nitro,
- ou bien un radical hétérocyclique choisi dans le groupe constitué par les radicaux indolyle, pyridyle, pipéridinyle, quinolyle et thiazolyle,
- ou bien un radical arylalkyle renfermant jusqu'à 18 atomes de carbone éventuellement substitué sur le noyau aryle par un ou plusieurs des substituants indiqués ci-dessus lorsque $R_1$ représente un radical aryle et

soit $R_2$ représente : - ou bien un radical

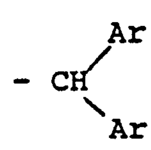

dans lesquels Ar et Ar′ identiques ou différents représentent un radical aryle renfermant jusqu'à 14 atomes de carbone éventuellement substitué par un ou plusieurs des substituants indiqués ci-dessus pour $R_1$, ou un radical hétérocyclique choisi dans le groupe constitué par les radicaux pyridyle, indolyle et thiazolyle,

- ou bien un radical

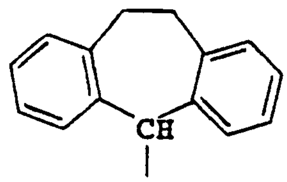

dans lequel les noyaux phényle peuvent être substitués par un ou plusieurs des substituants indiqués ci-dessus pour $R_1$,
et $R_3$ représente un atome d'hydrogène,
soit $R_2$ et $R_3$ forment ensemble avec l'atome d'azote, auquel ils sont liés un cycle carboné à 5, 6 ou 7 chaînons contenant éventuellement un atome d'oxygène ou un second atome d'azote éventuellement substitué par un ou plusieurs des radicaux choisis dans le groupe constitué par les radicaux alkyle renfermant jusqu'à 8 atomes de carbone, les radicaux aryle renfermant jusqu'à 14 atomes de carbone, les radicaux

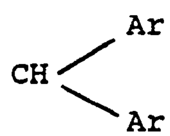

dans lesquels Ar et Ar′ conservent la même signification que précédemment,
et $R_4$ représente un atome d'hydrogène, un radical alkyle linéaire, ramifié ou cyclique renfermant jusqu'à 8 atomes de carbone, un radical arylalkyle renfermant jusqu'à 18 atomes de carbone éventuellement substitué par un ou plusieurs des substituants indiqués ci-dessus pour $R_1$, ou un radical

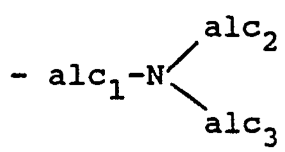

dans lesquels $alc_1$ représente un radical polyméthylène renfermant jusqu'à 8 atomes de carbone et $alc_2$ et $alc_3$ identiques ou différents représentent un radical alkyle linéaire, ramifié ou cyclique renfermant jusqu'à 8 atomes de carbone, sous toutes leurs formes isomères possibles ainsi que leurs mélanges.

2) Les composés de formule (I) tels que définis à la reven dication 1 dans lesquels $R_1$ représente un radical indolyle.

3) Les composés de formule (I) tels que définis à la revendication 1 ou 2 dans lesquels $R_2$ représente un radical :

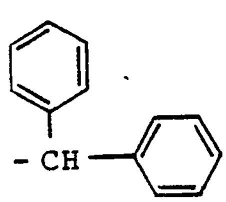

dans lequel les noyaux phényles sont éventuellement substitués par un ou plusieurs des substituants énumérés ci-dessus pour $R_1$.

4) Les composés de formule (I) tels que définis à la revendication 3 dans lesquels $R_2$ représente le radical :

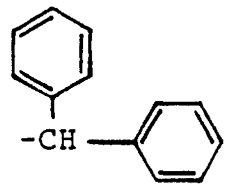

5) Les composés de formule (I) tels que définis à la revendication 3 dans lesquels $R_2$ représente l'un des radicaux suivants :

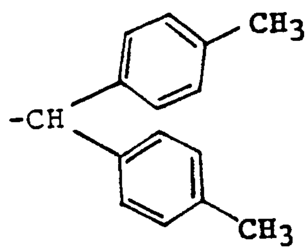

,

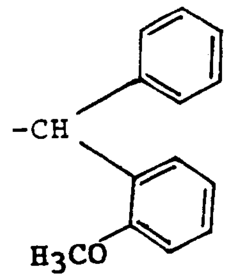

, et

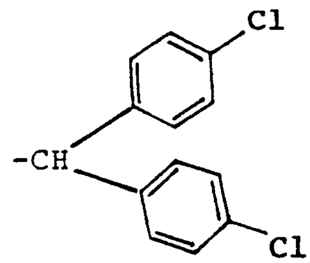

6) Les composés de formule (I) tels que définis à la revendication 1 ou 2 dans lesquels $R_2$ représente un radical :

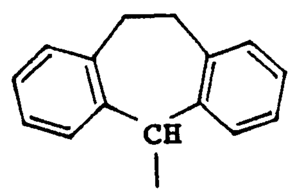

7) Les composés de formule (I) tels que définis à l'une quelconque des revendications 1 à 6, dans lesquels $R_4$ représente un atome d'hydrogène.

8) Les composés de formule (I) tels que définis à l'une quelconque des revendications 1 à 7 de configuration S au niveau du carbone en 4.

9) Les composés de formule (I) tels que définis à la revendication I dont les noms suivent :

**- Acide (S)-4-[[(1H-indol-2-yl) carbonyl] amino] 5-[[bis(4-méthyl phényl) méthyl] amino] 5-oxo pentanoïque**

**- Acide (S)-4-[[(1H-indol-2-yl) carbonyl] amino] 5-[(2-méthoxy phényl) phényl méthylamino] 5-oxo pentanoïque**

**- Acide (S)-5-[[bis(4-chloro phényl) méthyl] amino] 5-oxo 4-[[(1H-indol-2-yl) carbonyl] amino] pentanoïque**

**- Acide (S)-5-[(10,11-dihydrodibenzo[a,d]cyclohepten-5-yl) amino] 4-[[(1H-indol-2-yl) carbonyl] amino] 5-oxo pentanoïque.**

10) A titre de médicaments, les composés de formule (I) tels que définis à l'une quelconque des revendications 1 à 8.

11) A titre de médicaments, les composés de formule (I) tels que définis à la revendication 9.

12) Les compositions pharmaceutiques renfermant comme principe actif au moins un médicament défini à la revendication 10 ou 11.

13) Procédé de préparation des composés de formule (I) tels que définis à l'une quelconque des revendications 1 à 9, caractérisé en ce que l'on soumet un composé de formule (II) :

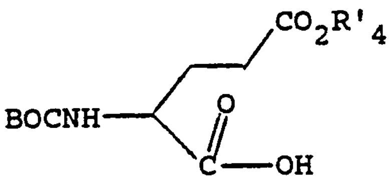

(II)

dans laquelle BOC représente le radical (1,1-diméthyl éthoxy) carbonyle et $R'_4$ a la même signification que $R_4$ à l'exception de la valeur hydrogène, à l'action d'un composé de formule (III) :

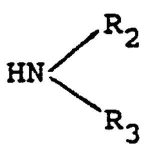

(III)

dans laquelle $R_2$ et $R_3$ conservent leur signification précédente pour obtenir le composé de formule (IV) :

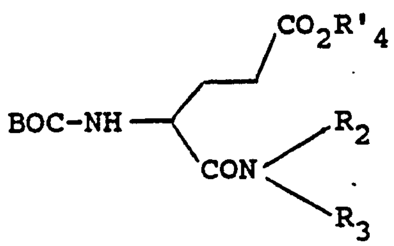

(IV)

que l'on soumet à l'action d'un agent de déblocage de la fonction amine, plus soumet le produit résultant à l'action d'un acide ou dérivé d'acide de formule (V) :

$R_1CO_2H$ (V)

dans laquelle $R_1$ conserve sa signification précédente pour obtenir le composé de formule ($I_A$) :

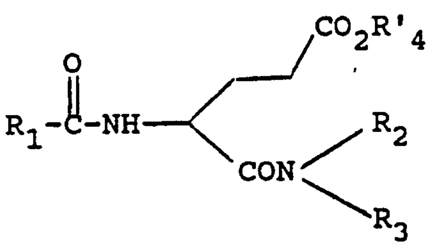

($I_A$)

que l'on soumet si désiré à l'action d'un agent de clivage de la fonction acide pour obtenir le composé de formule ($I_B$) :

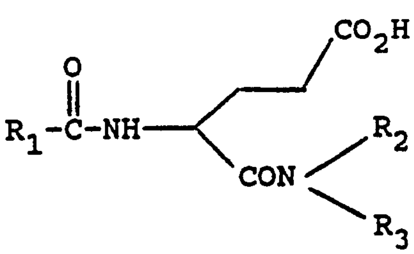

($I_B$)

14) Variante du procédé selon la revendication 13 caractérisé en ce que l'on soumet un composé de formule (VI) :

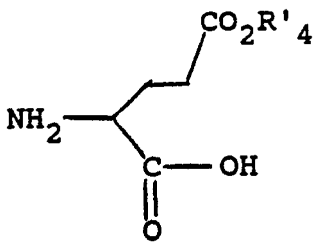

(VI)

dans laquelle $R'_4$ a la signification indiquée précédemment à l'action d'un acide ou d'un dérivé d'acide de formule (V) :

$R_1CO_2H$ (V)

dans laquelle $R_1$ a la signification indiquée précédemment pour obtenir le composé de formule (VII) :

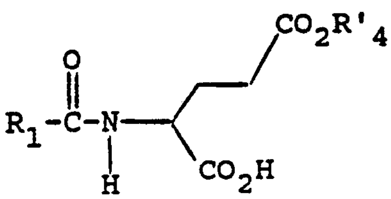

(VII)

que l'on soumet à l'action d'un composé de formule (III) :

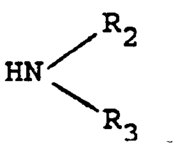

(III)

dans laquelle $R_2$ et $R_3$ ont la signification indiquée précédemment pour obtenir le composé de formule ($I_A$) correspondant que l'on soumet si désiré, à l'action d'un agent de clivage de la fonction acide pour obtenir le composé de formule ($I_B$), ($I_A$) et ($I_B$) étant définis comme précédemment.

15) A titre de produits chimiques nouveaux, les composés de formule (IV) et (VII) définis aux revendications 13 et 14.

Office européen des brevets

# RAPPORT DE RECHERCHE EUROPEENNE

Numero de la demande

EP 90 40 0416

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EUR. J. MED. CHEM. - CHIM. THER., vol. 21, no. 1, 1986, pages 9-20; F. MAKOVEC et al.: "New glutamic and aspartic derivatives with potent CCK-antagonistic activity"<br>* Page 13, composés 62,63; page 14 *<br>--- | 1,10 | C 07 D 209/42<br>C 07 D 213/82<br>C 07 D 215/54<br>C 07 D 211/60<br>C 07 C 237/06<br>A 61 K 31/40<br>A 61 K 31/44<br>A 61 K 31/47<br>A 61 K 31/445<br>A 61 K 31/195 |
| X | GB-A-2 160 869 (ROTTA RESEARCH LABORATORIUM SpA)<br>* Page 1, revendication 1 *<br>----- | 1,10 | |
| | | | **DOMAINES TECHNIQUES RECHERCHES (Int. Cl.5)** |
| | | | C 07 D 209/00<br>C 07 D 213/00<br>C 07 D 215/00<br>C 07 D 211/00<br>C 07 C 237/00 |

Le présent rapport a été établi pour toutes les revendications

| Lieu de la recherche | Date d'achèvement de la recherche | Examinateur |
|---|---|---|
| LA HAYE | 17-05-1990 | VAN BIJLEN H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)